# EUROPEAN PATENT SPECIFICATION

(11) **EP 0 956 868 B1**
(45) Date of publication and mention of the grant of the patent: **16.03.2005**
(21) Application number: 99303677.1
(22) Date of filing: 11.05.1999
(51) Int. Cl.: A61L 9/14

(54) **Air freshener**
Lufterfrischer
Rafraîchisseur d'air

(30) Priority: 12.05.1998 GB 9810136
(43) Date of publication of application: 17.11.1999
(73) Proprietor: Dudley Industries Ltd., Lytham, Lancs. FY8 5AT (GB)
(72) Inventor: Fielding, Robert Michael George, Lytham, St Annes, Lancs FY8 3HX (GB)
(74) Representative: Powell, Stephen David

(56) References cited:
- FR-A- 1 473 952
- FR-A- 2 454 810
- FR-A- 2 670 568
- GB-A- 398 943
- GB-A- 2 115 286
- GB-A- 2 236 482
- GB-A- 2 277 266
- US-A- 5 016 781

## Description

This invention relates to air freshening units for automatically releasing air freshener fragrances.

Known automatic air fresheners comprise a storage canister for containing the fragrant substance before it is released. Different fragrant substance release cycles lead to variable usage rates of the substance in the canister, making it difficult to predict when replacement will be necessary. As a result, canisters are frequently replaced too early with an amount of fragrant substance remaining. On other occasions, canisters become exhausted some time before replacement occurs.

The document FR-A-1473952 describes an air freshener allowing a selection of fragrances.

This invention seeks to provide an improved air freshener unit.

According to a first aspect of the present invention there is provided an air freshener unit for dispensing a fragrant substance comprising first and second substance sources each of which is independently replaceable characterised in that the air freshener unit comprises means for detecting when the first fragrant substance source is spent, and means for switching, in response to said detecting means, the release of air freshener to the second fragrant substance source.

Such an arrangement has the advantage of avoiding waste, while ensuring that the air freshener unit is always in operation.

In preferred embodiments, each source comprises a canister fitted with a controllable release valve mechanism. Each release valve mechanism is preferably solenoid operated and actuated by a control circuit including timer means. The timer means may comprise a simple timing device or a programmable real time clock.

Preferred units also comprise sensor means for determining when a canister is empty and indicating to the control circuit to switch between modes of operation using different canisters. Resettable indicator means enable the operator to quickly identify and replace spent canisters, e.g. during a periodic check.

According to a second aspect of the present invention, there is provided a method of releasing air freshener including the steps of:
controllably releasing a fragrant substance from a first fragrant substance source, detecting when the first fragrant substance source is spent, indicating that the first fragrant substance source is spent and automatically switching the release of air freshener to a second fragrant substance source.

To ensure the continuous release of air freshener, the first source is replaced after switching to the second source but before the second source is spent.

An embodiment of the present invention will now be described, by way of example only, with reference to the accompanying drawings of which:
Fig.1 shows a sectional front view of a preferred air freshener unit;
Fig.2 shows a sectional top view of the air freshener unit of Fig.1;
Fig.3 shows a sectional side view of the air freshener unit of Fig.1; and
Fig.4 shows a schematic representation of a modified air freshener unit.

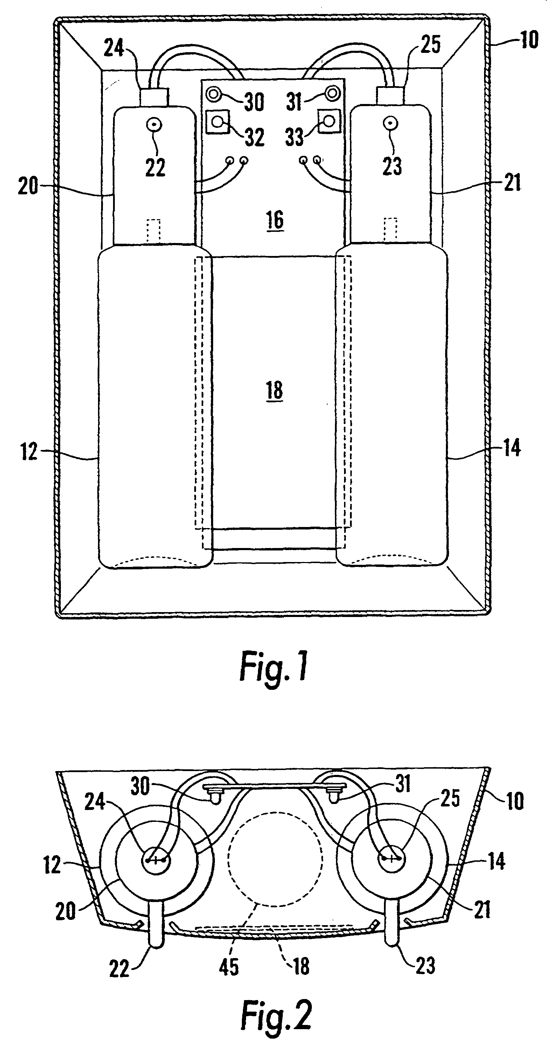

Referring to Figs. 1-3 of the drawings, the air freshener unit comprises a casing 10, first 12 and second 14 independent pressurised canisters for containing fragrant substances, an electronic control circuit 16 and a solar panel-type photoelectric cell device 18.

Each canister 12,14 is fitted with a solenoid operated release valve mechanism 20,21 supplying a separate spray outlet nozzle 22,23. Each release valve mechanism 20,21 includes a thermistor-type sensor built into the canister head 24,25.

The circuit 16 is mounted vertically and supplied with power generated by the photoelectric cell device 18 also mounted vertically towards the front of the casing. The circuit 16 includes timer means for selecting a time delay between operations of the release valve mechanism 20,21 to give delays of half, one or two hours. The circuit 16 also incorporates two "canister empty" indicator LEDs 30,31, one for each canister 12,14, and reset buttons 32,33 used to cancel the LEDs when an empty canister is replaced.

In use, two full canisters 12,14 are inserted into the air freshener unit and one or more of the reset means is activated to start operation. The unit operates by releasing the fragrant substance from the first canister 12 to the external atmosphere via release valve mechanism 20 and spray outlet nozzle 22. The timing of the release cycles is controlled by circuit 16 and the incorporated timer means. While the first canister 12 is operating normally, the thermistor-type sensor in the canister head outlet 24 senses a drop in temperature associated with the expansion of the fragrant fluid as it is expelled under pressure each time the release valve mechanism 20 operates. As the canister 12 becomes progressively more empty, this temperature drop diminishes and eventually falls below the detection threshold of the thermistor sensor. Reaching this "no detection" threshold is taken as an indication that the canister 12 is empty and the control circuit 16 switches over to operation using the second canister 14. The "empty indicator" LED 32 associated with the exhausted canister 12 begins to flash at approximately 10 second intervals and continues until the reset button is pressed when the canister 12 is replaced.

Between exhaustion of canister 12 and its replacement, the unit continues to operate normally by releasing the fragrant substance from the second canister 14 through release valve mechanism 21 and outlet nozzle 23. The empty canister 12 can be replaced at any time without interrupting the cycles of fragrant substance release from canister 14.

When the canister 14 becomes empty, the thermistor sensor in the second canister head 25 causes the control circuit 16 to transfer operation back to the newly replenished canister in the place of the first 12 and the second "empty indicator" LED 31 flashes until reset. The spent second canister 14 can then be replaced in a similar manner to the first without interrupting the operation of the unit.

Alternate replacement of the empty canister during a mode of operation dependent on the other can continue as necessary to maintain continuous operation. Allowing the unit to operate in the manner described allows greater freedom in source replenishment scheduling and removes uncertainty when different fragrant release cycles are employed. All canisters 12,14 are completely empty when they are removed, reducing waste and avoiding the need to dispose of partially spent canisters.

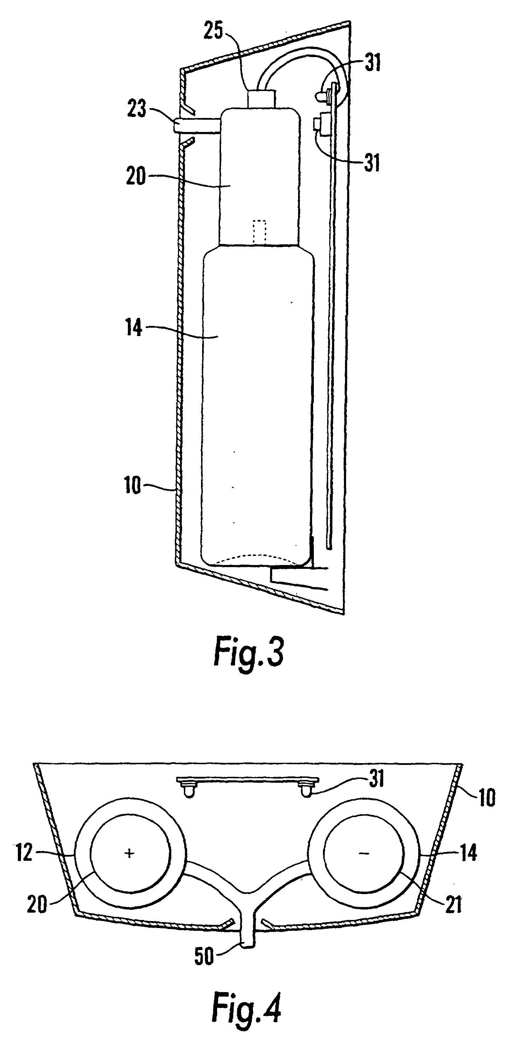

In modified versions of the air freshener unit, the configurations/orientations of the various components are different. For example, the circuit 16 and/or canisters 12,14 are mounted horizontally within the casing 10. In the embodiment shown in Fig.4, the release valve mechanisms 20,21 of the first and second canisters 12,14 supply a common outlet nozzle 50.

In other modified versions of the unit, the power supply for the control circuit 16 and other electronic components is provided by one or more batteries (e.g. 3 volt - x 'AA' or 'C' batteries) or a combination of batteries and photoelectric cell devices. To ensure overall compactness of the unit, the battery or batteries may be accommodated between the canisters 12,14 in a position indicated by reference numeral 45 in Fig.2.

In another modified version, the unit incorporates a real time clock having selectable/customisable programmes defining a number of predetermined operating cycles. For example, there may be options to have the release cycle operating 24 hours per day and 7 days per week or from 6am to 6pm between Monday and Friday only. In this way, unnecessary use can be avoided and waste reduced further.

In other modified versions detection means other than thermistor-type sensors may be used to sense when the level of fragrant substance inside a canister is running out. For example, fluid pressure measurement means, fluid flow rate measurement means or other suitable means could be used.

For some applications it may be preferable to have more than two replaceable canisters. A particularly advantageous feature of such a unit is that even fewer canister replacement visits are necessary.

The reset buttons 32,33 can alternatively be conventional switches, touch pads or any other suitable means. For other applications it is preferred that the empty indicator" LEDs are reset automatically as the canisters are replaced. For example, an electrical contact can be made/broken each time the casing 10 is replaced/removed or each time a canister 12,14 is replaced/removed.

## Claims

1. An air freshener unit for dispensing a fragrant substance comprising first and second substance sources (12, 14) each of which is independently replaceable **characterised in that** the air freshener unit comprises means for detecting (24) when the first fragrant substance source (12) is spent, and means (16) for switching, in response to said detecting means, the release of air freshener to the second fragrant substance source (14).

2. An air freshener unit according to claim 1, wherein the fragrant substance sources (12, 14) include controllable release means in the form of a solenoid operated valve device (20, 21), and the air freshener unit further includes control means (16) for controlling the valve device.

3. An air freshener unit according to claim 2, wherein the control means includes timer means, sensor means (24, 25), and indicator means (30, 31) for respectively selecting a time delay, for sensing when the first or second substance sources is spent, and for indicating that a source is spent.

4. An air freshener unit according to claim 3, wherein the sensor means is a thermistor-type sensor (24, 25).

5. An air freshener unit according to claim 3, wherein the sensor means is a fluid pressure measurement means, or a fluid flow rate measurement means.

6. An air freshener unit according to any of claims 3 to 5, wherein the timer means is programmable.

7. An air freshener unit according to any of claims 3 to 6, wherein the indicator means (30, 31) are resettable.

8. A method of releasing air freshener including the steps of:
controllably releasing a fragrant substance from a first fragrant substance source (12), detecting when the first fragrant substance source is spent, indicating that the first fragrant substance source is spent and automatically switching the release of air freshener to a second fragrant substance source (14).

9. A method according to claim 8, wherein the first source (12) is replaced after switching to the second source (14) but before the second source is spent.

10. A method according to claim 9, wherein, when the second source (14) is spent, the release of air freshener is automatically switched to a replacement source (12).

## Patentansprüche

1. Luftauffrischungs-Vorrichtung für das Abgeben einer Duftsubstanz, umfassend eine erste und eine zweite Substanzquelle (12, 14), von denen jede eigenständig austauschbar ist, **dadurch gekennzeichnet, dass** die Luftauffrischungs-Vorrichtung Mittel zum Detektieren (24) des Zeitpunktes, zu dem die erste Duftsubstanzquelle (12) erschöpft ist, und Mittel (16) zum Umschalten der Freisetzung von Luftauffrischer auf die zweite Duftsubstanz-quelle (14) als Antwort auf die Detektions-Mittel, umfasst.

2. Luftauffrischungs-Vorrichtung nach Anspruch 1, wobei die Duftsubstanzquellen (12, 14) steuerbare Freisetzungs-Mittel in Form einer elektromagnetisch betätigten Ventilvorrichtung (20, 21) umfassen, und die Luftauffrischungs-Vorrichtung ferner Steuer-Mittel (16) zum Steuern der Ventilvorrichtung umfasst.

3. Luftauffrischungs-Vorrichtung nach Anspruch 2, wobei das Steuer-Mittel Zeitgeber-Mittel, Sensor-Mittel (24, 25) und Anzeige-Mittel (30, 31) zum Wählen einer Zeitverzögerung, zum Detektieren des Zeitpunktes, zu dem die erste oder zweite Substanzquelle erschöpft ist, bzw. zum Anzeigen, dass eine Quelle erschöpft ist, umfasst.

4. Luftauffrischungs-Vorrichtung nach Anspruch 3, wobei das Sensor-Mittel ein Sensor vom Thermistor-Typ (24, 25) ist.

5. Luftauffrischungs-Vorrichtung nach Anspruch 3, wobei das Sensor-Mittel ein Flüssigkeitsdruckmesser oder ein Flüssigkeits-Durchflussmesser ist.

6. Luftauffrischungs-Vorrichtung nach einem der Ansprüche 3 bis 5, wobei das Zeitgeber-Mittel programmierbar ist.

7. Luftauffrischungs-Vorrichtung nach einem der Ansprüche 3 bis 6, wobei die Anzeige-Mittel (30, 31) rücksetzbar sind.

8. Verfahren zur Freisetzung von Luftauffrischer, umfassend die folgenden Schritte:
Gesteuertes Freisetzen einer Duftsubstanz von einer ersten Duftsubstanzquelle (12); Detektieren wenn die erste Duftsubstanzquelle erschöpft ist; Anzeigen, dass die erste Duftsubstanzquelle erschöpft ist; und automatisches Umschalten der Freisetzung von Luftauffrischer auf eine zweiten Duftsubstanzquelle (14).

9. Verfahren nach Anspruch 8, wobei die erste Substanzquelle (12) nach dem Umschalten auf die zweite Substanzquelle (14) ausgetauscht wird, aber bevor die zweite Substanzquelle erschöpft ist.

10. Verfahren nach Anspruch 9, wobei, wenn die zweite Quelle (14) erschöpft ist, die Freisetzung von Luftauffrischer automatisch auf eine Austauschquelle (12) umgeschaltet wird.

## Revendications

1. Unité d'assainissement d'air destinée à diffuser une substance parfumée comprenant des première et seconde sources de substance (12, 14), chacune d'elles pouvant être remplacée de manière indépendante, **caractérisée en ce que** l'unité d'assainissement d'air comprend un moyen (24) destiné à détecter que la première source de substance parfumée (12) est épuisée, et un moyen (16) destiné à commuter, en réponse audit moyen de détection, la libération d'unité d'assainissement d'air à partir de la seconde source de substance parfumée (14).

2. Unité d'assainissement d'air selon la revendication 1, dans laquelle les sources de substance parfumée (12, 14) comportent un moyen de libération pouvant être commandé sous la forme d'un dispositif formant une vanne (20, 21), actionné par solenoïde et l'unité d'assainissement d'air comporte, en outre, un moyen de commande (16) destiné à commander le dispositif formant vanne.

3. Unité d'assainissement d'air selon la revendication 2, dans laquelle le moyen de commande comporte un moyen formant minuteur, un moyen de détection (24, 25) et des moyens formant indicateur (30, 31) destinés respectivement à sélectionner une durée, à détecter que la première ou la seconde source de substance est épuisée et à indiquer qu'une source est épuisée.

4. Unité d'assainissement d'air selon la revendication 3, dans laquelle le moyen de détection est un capteur du type thermistance (24, 25).

5. Unité d'assainissement d'air selon la revendication 3, dans laquelle le moyen de détection est un moyen de mesure de pression fluide ou un moyen de mesure de débit de fluide.

6. Unité d'assainissement d'air selon l'une quelconque des revendications 3 à 5, dans laquelle le moyen formant minuteur est programmable.

7. Unité d'assainissement d'air selon l'une quelconque des revendications 3 à 6, dans laquelle les moyens formant indicateur (30, 31) peuvent être réinitialisés.

8. Procédé de libération d'agent d'assainissement d'air comportant les étapes de:
libération de manière contrôlée d'une substance parfumée à partir d'une première source de substance parfumée (12), détection du fait que la première source de substance parfumée est épuisée, indication du fait que la première source de substance parfumée est épuisée et de commutation automatique de la libération d'agent d'assainissement d'air à partir d'une seconde source de substance parfumée (14).

9. Procédé selon la revendication 8, dans lequel la première source (12) est remplacée après commutation sur la seconde source (14), mais avant que la seconde source soit épuisée.

10. Procédé selon la revendication 9, dans lequel, lorsque la seconde source (14) est épuisée, la libération d'agent d'assainissement d'air est commutée automatiquement sur une source de remplacement (12).
